# FASCICULE DE BREVET EUROPEEN

(11) **EP 1 572 220 B1**
(45) Date de publication et mention de la délivrance du brevet: **13.02.2008**
(21) Numéro de dépôt: 02781622.2
(22) Date de dépôt: 11.12.2002
(51) Int. Cl.: A61K 36/48, A61P 31/18

(54) **PROPRIETES ANTIVIRALES DES EXTRAITS DE DICHROSTACHYS GLOMERATA**
ANTIVIRALE WIRKUNGEN VON DICHROSTACHYS GLOMERATA EXTRAKTEN
ANTIVIRAL PROPERTIES OF EXTRACTS OF DICHROSTACHYS GLOMERATA

(43) Date de publication de la demande: 14.09.2005
(73) Titulaire: Agon, Achidi Valentin, Cotonou Houeyiho (BJ); Kinnoudo, Célestin, Bohicon (BJ)
(72) Inventeur: Agon, Achidi Valentin, Cotonou Houeyiho (BJ); Kinnoudo, Célestin, Bohicon (BJ)
(74) Mandataire: Laget, Jean-Loup
(86) Numéro de dépôt international: PCT/IB2002/005285
(87) Numéro de publication internationale: WO 2004/052384

(56) Documents cités:
- WO-A-00/01398
- US-A- 3 089 817
- KUDI A C ET AL: "Antiviral activity of some Nigerian medicinal plant extracts" JOURNAL OF ETHNOPHARMACOLOGY 1999 IRELAND, vol. 68, no. 1-3, 1999, pages 289-294, XP002247211 ISSN: 0378-8741
- KAMBIZI L ET AL: "An ethnobotanical study of plants used for the treatment of sexually transmitted diseases (njovhera) in Guruve District, Zimbabwe." JOURNAL OF ETHNOPHARMACOLOGY, vol. 77, no. 1, septembre 2001 (2001-09), pages 5-9, XP002247212 ISSN: 0378-8741

## Description

### DOMAINE TECHNIQUE : PHARMACIE

Nous avons découvert une plante dont l'extraction par décoction à 100°C sert à fabriquer de médicament antiretroviral d'efficacité immédiate en monothérapie sur le VIH/SIDA. Le traitement qu'offre cette médication consiste à baisser la charge virale (nombre de copies de ARN viral par millilitre de sang) jusqu'à la rendre indétectable et à restaurer le système immunitaire détruit par le VIH et par conséquent le patient retrouve sa parfaite santé. L'origine naturelle de cette substance active fait d'elle une médication qui échappe à la possibilité de développement de résistance de la part de VIH , (VIRUS D'IMMUNODEFICIENCE HUMAINE) RESPONSABLE DE LA MALADIE DU SIDA (SYNDROME D'IMMUNODEFICIENCE ACQUISE) découvert par le Professeur LUC MONTAGNIER en 1981

La technique que nous proposons est la décoction à 100°C qui s'utilise depuis longtemps en pharmacopée : la plante est baignée dans l'eau portée à ébullition pendant 45 minutes et laissées pendant 6 heures.

Nous allons exposer de la connaissance de la plante ressource jusqu'à l'obtention du principe actif que nous dénommons : « APIVIRINE »

### TECHNIQUE ANTERIEURE

De façon générale si une industrie pharmaceutique a connaissance des vertus thérapeutiques d'une plante, les procédés et les techniques utilisés pour l'extraction du principe actif ou substance active varient selon les objectifs. Par exemples par la distillation on peut extraire les huiles essentielles d'une plante, on peut utiliser l'eau ou l'alcool pour extraire des principes actifs selon que ce dernier soit soluble dans l'eau ou dans l'alcool.

D'après la publication scientifique de Kudi A. C. et Myint S. H., « Antiviral activity of some Nigerian medicinal plant extracts », JOURNAL OF ETHNOPHARMACOLOGY 1999 IRELAND, vol. 68, N°1-3, 1999, pages 289-294, des extraits de la plante médicinale Dichrostachys glomerata ont été utilisés pour traiter des infections virales par le poliovirus, l'astrovirus, l'herpes simplex virus 1 (HSV1), le HSV équin et le parvovirus bovin et canin.

En outre, d'après la publication scientifique de Kambizi L. et Afolayan A.J., « AN ethnobotanical study of plants used for the treatment of sexually transmitted disease (njovhera) in Guruve District, Zimbabwe », JOURNAL OF ETHNOPHARMACOLOGY, vol. 77, N°1, 1er septembre 2001, pages 5-9, des extraits de la plante médicinale Dichrostachys cinerea ont été utilisés pour traiter des maladies sexuellement transmissibles d'origine bactériologique.

Enfin, le document US 3 089 817 révèle l'utilisation d'extraits de la plante médicinale Dichrostachys glomerata comme agent hypotensif.

### 1-LA PLANTE

1- 1-Nom scientifique : Dichrostachys glomerata (dans la langue locale FON (langue nationale du BENIN le nom est)
1- 2- BADAWIN
1- 3- Nom en Français : mimosa clochette
1- 4-Famille botanique : mimosacée
1- 5-Source botanique : plante tropicale répartie dans toute l'Afrique de l'ouest.
1- 6-La substance extraite de la plante est acide
1- 7-Chémotype : 0 à 500 mètres d'altitude en région tropicale (Altitude à laquelle la plante est très riche en cette propriété thérapeutique et non toxique selon nos sites de recherche du Sud au Nord BENIN)
1- 8-Maturité : La plante adulte est utile à tout moment , seules les jeunes pousses du début des saisons pluvieuses doivent être récoltées deux mois au moins après les premières pluies de l'année.
1- 9-Partie utile de la plante : La partie aérienne de la plante (le houppier ou le feuillage) sauf les branches de diamètres supérieur à deux centimètres.
   NB : Le rapport naturel quantité de feuilles / branche pendant la saison pluvieuse doit être respecté (la plante est bien touffue en saison pluvieuse et c'est le contraire en sécheresse)
1- 10-Description de la plante : plante caractérisée par des épines bizarres bien prononcées, ses feuilles sont très petites, ses fleurs attirent les abeilles, elle est une légumineuse qui donne des gousses repliée sur elle même.
1- 11-Reproductibilité à grande échelle : Cette plante est facilement reproductible à grande échelle , au début des saisons pluvieuses les jeunes pousses qui ressortent des racines sont très nombreuses et sont faciles à être transplantées
1- 12-TOXICITE : cette plante médicinale n'est nullement toxique

### 2-LA RECOLTE ET LE STOCKAGE

Toutes les branches de la plante (arbrisseau qui devient à long terme un arbuste) garnies de feuilles vertes sont bonnes à être récoltées. Les feuilles qui commencent à jaunir sont à écarter (effet de la sécheresse). Une fois la récolte faite, le tout est stocké dans un bâtiment à l'abri des rayons solaires, étalé sur le sol cimenté ou en béton, l'épaisseur de l'étalage ne doit pas dépasser 15 centimètres sinon les feuilles pourrissent. Aussi longtemps que ces conditions sont respectées, les feuilles , bien que séchées, conservent leur propriété thérapeutique.

L'extraction peut se faire aussitôt après récolte, si cela est possible c'est très bien car la plante cède facilement son principe actif.

### 3-L'EXTRACTION DU PRINCIPE ACTIF :APIVIRINE

### 3-1-METHODE : DECOCTION

### 3- 1-1-La pesée de la plante récoltée

La quantité totale à extraire doit être pesée et bien notée. Cette étape est très importante car la qualité (la non toxicité et la puissance antiretrovirale : la force de frappe antivirale) du principe actif dépends du poids total de la plante bouillie. Pendant la pesée les branches de plus de deux centimètres doivent être retirées, sinon elles influencent le poids total et fausse le rapport POIDS TOTAL/QUALITE du principe actif.

### 3- 1- 2-Extraction proprement dite

### 3-1-2-1-Première phase : la cuisson

L'eau doit surnager les feuilles et une fois l'ébullition est atteinte, elle doit durer quarante cinq (45) minutes, après ce temps on complète l'eau (à cause de la perte d'eau due à l'évaporation) et l'intensité du chauffage doit être maintenue jusqu'à une nouvelle ébullition après laquelle la source du chauffage doit être coupée et les feuilles sont laissées dans l'eau de cuisson pendant 6 heures.

### 3- 1-2-1-Deuxième phase : retrait des feuilles, rinçage et filtration

On retire les feuilles, on rince, on rajoute le liquide issu du rinçage à celui de l'extraction, et on utilise ce liquide pour faire une nouvelle extraction et au tant de fois que l'on veut. On complète l'eau pour qu'elle surnage les feuilles. Le rinçage doit être rigoureusement bien fait pour récupérer le maximum de la substance active. on filtre le liquide de la dernière extraction pour passer à la déshydratation. NB : L'avantage d'utiliser le même liquide pour faire de nouvelles extractions est qu'on a une forte concentration du principe actif dans peu d'eau et la déshydratation prend moins de temps.

### 3- 1-2-1-Troisième phase : l'obtention du principe actif « APIVIRINE »

### 3-1-2-1-1-Méthode : la déshydratation

Tout le liquide issu de l'extraction obtenu après filtration est soumis à un chauffage en vue de la déshydratation complète, le but est d'obtenir le principe actif sous sa forme la plus pure. On fait évaporer le liquide jusqu'à obtenir une substance noire de PH situé entre 3 et 4 et de taux d'humidité inférieur à 10 pour cent (mesure faite avec un réfractomètre). Vers la fin, le chauffage doit être doux et maîtrisé de telle façon que la substance ne sera pas carbonisée. La substance se solidifie sans aucune intervention particulière après refroidissement si la déshydratation est parfaite.

Une précision importante: cent kilogrammes (100KG) de plante traitée donne 3100 grammes de APIVIRINE pure de PH situé entre 3 et 4 et taux d'humidité inférieur à 10 pour cent. Ce pur concentré d'apivirine se conserve sans autre précaution et peut être expédié partout dans le monde sans difficulté. Si le taux d'humidité n'est pas respecté la substance va moisir et cela affecte la qualité du produit fini.

Dans l'eau chaude apivirine se dissout facilement et peut être exploitée aisément.
NB : Au cours de la déshydratation le liquide a tendance à monter et à remplir son contenant pour le déborder. Une surveillance est de rigueur pour empêcher ce débordement qui fait perdre de molécules et qui diminue la quantité du principe actif qui doit s'extraire de la quantité totale de plante.

### 3-2-Précision importante

Le matériel qui sert à la macération et à la déshydratation doit être inoxydable

### 4 - LA TRANSFORMATION D'APIVIRINE EN PRODUIT FINI : MEDICAMENT

Une fois le principe actif sous sa forme pure est obtenue, n'importe qu'elle industrie pharmaceutique peut l'exploiter et le transformer en médicament sous diverse forme : sirop, comprimé, gélule, ampoule buvable, etc.

### 5 - CLASSES PHARMACOTHERAPEUTIQUES

### 5-1- LE VIH/SIDA

Cette substance antivirale a une force de frappe exceptionnelle sur le VIH, l'effet est immédiat si la dose journalière est à la taille de la charge virale du patient. C'est un principe actif qui se classe parmi les antiretroviraux ANTIVIH/SIDA et particulièrement d'origine naturelle.

### 5-1-1- Posologie

Une précision très importante pour la posologie pour un patient adulte : la dose journalière d'apivirine pour un adulte est la concentration moléculaire issue de l'extrait de 970 grammes de plante soit 30 grammes d'apivirine réparties en trois prises journalières après les repas, la dose par prise est donc 10 grammes, la dose, au-delà de cette limite, rend les selles dures pour certaines personnes bien qu'ayant une force de frappe antivirale supérieure. On peut accepter dépasser cette dose journalière sans toutefois atteindre 15 grammes par prise, cette posologie (10 à 15g/prise) est pour les traitements d'attaque ou pour arrêter immédiatement les diarrhées persistantes liées au multiple infections ou surtout pour détruire le maximum de virus en peu de jours en vue d'obtenir une charge virale indétectable en moins d'un mois.

Toute femme en état de grossesse ou toute nourrice peut consommer ce principe actif sans inconvénient

La posologie pour enfant dépend de la tolérance observée à son niveau par le médecin traitant, la dose conseillée est nettement inférieure au 1/3 de celle des adultes et cela est en fonction de son âge.

### 5-1-2- Effets indésirables

Cette substance rend les selles dures pour certaines personnes

### 5-1-3-Conseils

Au cas où il y a des selles dures, l'usage de tout laxatif est recommandé, l'acidité du sirop d'apivirine fait que certaines personnes ont tendance à vomir juste après la prise, il suffit de diluer le sirop dans un démi verre d'eau pour éviter cet effet.

### 5-1-4-Durée de traitement

Le traitement doit être continu même si la charge virale du patient est indétectable (inférieur au seuil de sensibilité de l'appareil qui fait la charge virale, aujourd'hui ce seuil est 10 ou 20 ou 50 selon la performance de la machine) et sa santé parfaitement retrouvée, donc à vie selon nos connaissances d'aujourd'hui.

### 5-1-5-Développement de résistance de VIH est-il possible ?

Le VIH n'arrive pas à développer une résistance contre ce principe actif.

Cette substance étant cent pour cent naturelle (non synthétisée au laboratoire) garde de façon permanente sa propriété thérapeutique contre le VIH. L'origine naturelle de sa composition chimique lui confère cette puissance, ce n'est pas une molécule qui est dirigée contre le virus, mais un ensemble de molécules toutes antivirales capables d'inhiber l'infection à VIH. Certes cette composition varie dans le temps et selon les saisons mais demeure efficace sur les germes sensibles.

### 5-1-6- PARAMETRES BIOLOGIQUES QUI CONFIRMENT L'EFFICACITE ANTIRETROVERALE ANTIVIH DE APIVIRINE

### 5-1-6- 1-La charge virale

C'est l'examen médical qui détermine l'efficacité et la force de frappe antivirale de toute substance ayant effet sur VIH. Il révèle à l'instant T le nombre de virus existant. Cette analyse est reprise chaque mois ou tous les deux mois pour constater la baisse progressive du nombre de copie de ARN viral par millilitre de sang jusqu'au résultat indétectable

### 5-1-6- 1-La numération des lymphocytes CD4 ou T4

C'est l'examen médical qui permet de constater la restauration du système immunitaire du patient sous un traitement anti-VIH. Cette analyse est reprise tous les quatre ou six mois. C'est le comptage du nombre des cellules T par mm³ de sang.

## Revendications

1. Utilisation de la plante médicinale de nom scientifique Dichrostachys glomerata pour la fabrication d'un médicament antirétroviral destiné au traitement de l'infection par le virus d'immunodéficience humaine (VIH) agent causal du syndrome d'immunodéficience acquise (SIDA).

2. Utilisation de la plante médicinale de nom scientifique Dichrostachys glomerata selon la revendication 1, pour baisser la charge virale jusqu'à un résultat indétectable et restaurer le taux de lymphocytes CD4 (T4).

3. Utilisation de la plante médicinale de nom scientifique Dichrostachys glomerata selon l'une des revendications 1 et 2, sous forme d'un extrait de ladite plante médicinale.

4. Utilisation de la plante médicinale de nom scientifique Dichrostachys glomerata selon la revendication 3, dans laquelle ledit extrait de ladite plante médicinale est obtenu par le procédé de fabrication comportant au moins :
a. Une étape de décoction de la partie aérienne de la plante médicinale Dichrostachys glomerata dans l'eau, laquelle étape de décoction comprend :
• une première phase de cuisson à température d'ébullition préférentiellement pendant 45 minutes,
• une phase d'ajout d'un volume d'eau remplaçant le volume d'eau évaporé pendant la première phase de cuisson,
• une seconde phase de cuisson jusqu'à ébullition et
• une phase de repos sans chauffage préférentiellement pendant 6 heures ;
b. Une étape de séparation de la partie aérienne de la plante médicinale Dichrostachys glomerata du liquide issu de l'étape de décoction, laquelle étape de séparation comprend :
• le retrait la partie aérienne de la plante médicinale Dichrostachys glomerata du liquide issu de l'étape de décoction,
• un premier rinçage de la partie aérienne de la plante médicinale Dichrostachys glomerata par de l'eau de façon à obtenir un liquide issu du premier rinçage,
• le mélange dudit liquide issu de l'étape de décoction et dudit liquide issu du premier rinçage,
• un deuxième rinçage de ladite partie aérienne de la plante médicinale Dichrostachys glomerata par le produit issu dudit mélange de façon à former un liquide issu du deuxième rinçage,
• la filtration dudit liquide issu du dernier rinçage de façon à former un liquide filtré ;
c. Une étape de déshydratation dudit liquide filtré, laquelle étape de déshydratation comprend l'évaporation sous chauffage dudit liquide filtré de façon à obtenir ledit extrait de Dichrostachys glomerata, ladite évaporation sous chauffage étant maintenue jusqu'à ce que la couleur dudit extrait de Dichrostachys glomerata soit noire, que le pH dudit extrait de Dichrostachys glomerata soit situé entre 3 et 4, et que le taux d'humidité dudit extrait de Dichrostachys glomerata soit inférieur à 10%.

5. Utilisation de la plante médicinale de nom scientifique Dichrostachys glomerata selon la revendication 4, dans laquelle ledit extrait de ladite plante médicinale est obtenu par le procédé de fabrication dans lequel au moins un rinçage additionnel de ladite partie aérienne de la plante médicinale Dichrostachys glomerata est effectué après le deuxième rinçage de ladite partie aérienne de la plante médicinale Dichrostachys glomerata, chaque rinçage additionnel étant effectué à partir du liquide issu du rinçage précédent.

6. Utilisation de la plante médicinale de nom scientifique Dichrostachys glomerata selon l'une des revendications précédentes, dans laquelle ledit médicament est sous forme de sirop, comprimé, gélule ou ampoule buvable en monothérapie.

7. Utilisation de la plante médicinale de nom scientifique Dichrostachys glomerata selon l'une des revendications précédentes, dans laquelle ledit médicament est sous forme de prises pour un patient adulte qui permettent l'administration d'une dose journalière dudit extrait de 30 grammes répartie en trois prises.

8. Utilisation de la plante médicinale de nom scientifique Dichrostachys glomerata selon la revendication 6, dans laquelle la dose en extrait dans chaque prise n'excède pas 15 grammes et est préférentiellement égale à 10 grammes.

9. Utilisation de la plante médicinale de nom scientifique Dichrostachys glomerata selon l'une des revendications précédentes, dans laquelle ledit médicament est sous forme de prises pour un patient enfant qui permettent l'administration d'une dose journalière dudit extrait de 10 grammes répartie en trois prises.

10. Utilisation de la plante médicinale de nom scientifique Dichrostachys glomerata selon la revendication 9, dans laquelle la dose en extrait dans chaque prise est de 3,33 grammes.

## Claims

1. Use of the medicinal plant with scientific name Dichrostachys glomerata for the production of an antiretroviral medicinal product intended for the treatment of human immunodeficiency virus (HIV), the agent responsible for acquired immunodeficiency syndrome (AIDS).

2. Use of the medicinal plant with scientific name Dichrostachys glomerata according to claim 1, to lower viral load to an undetectable level and to restore the level of CD4 (T4) lymphocytes.

3. Use of the medicinal plant with scientific name Dichrostachys glomerata according to one of claims 1 and 2, in the form of an extract of said medicinal plant.

4. Use of the medicinal plant with scientific name Dichrostachys glomerata according to claim 3, in which said extract of said medicinal plant is obtained by a manufacturing process including at least:
a: A step of decoction of the aerial part of the medicinal plant Dichrostachys glomerata in water, with the decoction step including:
- an initial phase of cooking at boiling temperature, preferably for 45 minutes,
- a phase of addition of a volume of water to replace the volume of water evaporated during the initial cooking phase,
- a second phase of cooking to boiling, and
- a resting phase without heat, preferably lasting 6 hours;
b. A step of separation of the aerial part of the medicinal plant Dichrostachys glomerata from the liquid resulting from the decoction step, with this separation step including:
- removal of the aerial part of the medicinal plant Dichrostachys glomerata from the liquid resulting from the decoction step,
- an initial rinse of the aerial part of the medicinal plant Dichrostachys glomerata with water in order to obtain a liquid from the first rinse,
- mixing of said liquid resulting from the decoction step and of said liquid resulting from the first rinse,
- a second rinse of said aerial part of the medicinal plant Dichrostachys glomerata with the product resulting from said mixture in order to form a liquid resulting from the second rinse,
- filtration of said liquid resulting from the last rinse in order to form a filtered liquid;
c. A step of dehydration of said filtered liquid, with this dehydration step including evaporation under heat of said filtered liquid in order to obtain said extract of Dichrostachys glomerata, said evaporation under heat being maintained until the color of said extract of Dichrostachys glomerata is black, the pH of said extract of Dichrostachys glomerata is between 3 and 4, and the moisture level of said extract of Dichrostachys glomerata is lower than 10%.

5. Use of the medicinal plant with scientific name Dichrostachys glomerata according to claim 4, in which said extract of said medicinal plant is obtained by the production process in which at least one additional rinse of said aerial part of the medicinal plant is performed after the second rinse of said aerial part of the medicinal plant Dichrostachys glomerata, with each additional rinse being performed using the liquid resulting from the previous rinse.

6. Use of the medicinal plant with scientific name Dichrostachys glomerata according to any of the above claims, in which said medicinal product is in the form of a syrup, a tablet, a caplet or ampoule for oral administration, as monotherapy.

7. Use of the medicinal plant with scientific name Dichrostachys glomerata according to any of the above claims, in which said medicinal product is in the form of doses for an adult patient that allow administration of a daily dosage of said extract of 30 grams divided into three doses.

8. Use of the medicinal plant with scientific name Dichrostachys glomerata according to claim 6, in which the amount of extract in each dose does not exceed 15 grams and is preferably equal to 10 grams.

9. Use of the medicinal plant with scientific name Dichrostachys glomerata according to any of the above claims, in which said medicinal product is in the form of doses for a child patient which allow administration of a daily dosage of said extract of 10 grams divided into three doses.

10. Use of the medicinal plant with scientific name Dichrostachys glomerata according to claim 9, in which the amount of extract in each dose is 3.33 grams.

## Patentansprüche

1. Verwendung der Heilpflanze mit dem wissenschaftlichen Namen Dichrostachys glomerata für die Herstellung eines antiretroviralen Arzneimittels zur Behandlung der Infektion durch das humane Immunschwäche-Virus (HIV), Erreger des erworbenen Immundefektsyndroms (AIDS).

2. Verwendung der Heilpflanze mit dem wissenschaftlichen Namen Dichrostachys glomerata nach Anspruch 1, um die Viruslast bis zu einem nicht nachweisbaren Ergebnis zu senken und die CD4-Lymphozytenrate (T4) wiederherzustellen.

3. Verwendung der Heilpflanze mit dem wissenschaftlichen Namen Dichrostachys glomerata nach einem der Ansprüche 1 und 2 in Form eines Extrakts der besagten Heilpflanze.

4. Verwendung der Heilpflanze mit dem wissenschaftlichen Namen Dichrostachys glomerata nach Anspruch 3, bei der der besagte Extrakt der besagten Heilpflanze durch das Herstellungsverfahren erhalten wird, das mindestens umfasst:
a. Eine Dekoktionsetappe des oberirdischen Teils der Heilpflanze Dichrostachys glomerata in Wasser, wobei diese Dekoktionsetappe umfasst:
• eine erste Auskochphase bei Siedetemperatur vorzugsweise während 45 Minuten,
• eine Phase des Hinzufügens eines Wasservolumens, welches das während der ersten Auskochphase verdampfte Wasservolumen ersetzt,
• eine zweite Auskochphase bis zum Sieden und
• eine Ruhephase ohne Wärmezufuhr vorzugsweise während 6 Stunden;
b. Eine Trennungsphase des oberirdischen Teils der Heilpflanze Dichrostachys glomerata von der Flüssigkeit, die aus der Dekoktionsetappe stammt, wobei diese Trennungsphase umfasst:
• Die Entnahme des oberirdischen Teils der Heilpflanze Dichrostachys glomerata aus der Flüssigkeit, die aus der Dekoktionsetappe stammt,
• Eine erste Spülung des oberirdischen Teils der Heilpflanze Dichrostachys glomerata mit Wasser, so dass man eine Flüssigkeit enthält, die aus der ersten Spülung stammt,
• Die Mischung der besagten Flüssigkeit, die aus der Dekoktionsetappe stammt, mit der besagten Flüssigkeit, die aus der ersten Spülung stammt,
• Eine zweite Spülung des besagten oberirdischen Teils der Heilpflanze Dichrostachys glomerata durch das Produkt, das aus dieser besagten Mischung stammt, so dass eine Flüssigkeit gebildet wird, die aus der zweiten Spülung stammt.
• Die Filtrierung der besagten Flüssigkeit, die aus der letzten Spülung stammt, so dass eine gefilterte Flüssigkeit entsteht.
c. Eine Dehydratationsphase der besagten gefilterten Flüssigkeit, wobei diese Dehydratationsphase die Verdampfung unter einer Wärmequelle der besagten gefilterten Flüssigkeit umfasst, so dass man den besagten Dichrostachys glomerata-Extrakt erhält, wobei die besagte Verdampfung unter einer Wärmequelle so lange aufrechterhalten wird, bis die Farbe des besagten Dichrostachys glomerata-Extrakts schwarz ist, der pH-Wert des besagten Dichrostachys glomerata-Extrakts zwischen 3 und 4 liegt und der Feuchtigkeitsgehalt des besagten Dichrostachys glomerata-Extrakts unter 10 % beträgt.

5. Verwendung der Heilpflanze mit dem wissenschaftlichen Namen Dichrostachys glomerata nach Anspruch 4, bei der der besagte Extrakt der besagten Heilpflanze durch ein Herstellungsverfahren erhalten wird, bei dem mindestens eine zusätzliche Spülung des besagten oberirdischen Teils der Heilpflanze Dichrostachys glomerata nach der zweiten Spülung des besagten oberirdischen Teils der Heilpflanze Dichrostachys glomerata durchgeführt wird, wobei jede zusätzliche Spülung mit der Flüssigkeit durchgeführt wird, die aus der vorhergehenden Spülung stammt.

6. Verwendung der Heilpflanze mit dem wissenschaftlichen Namen Dichrostachys glomerata nach einem der vorausgehenden Ansprüche, bei der das Arzneimittel in Form von Saft, Tablette, Kapsel oder Trinkampulle als Monotherapie vorliegt.

7. Verwendung der Heilpflanze mit dem wissenschaftlichen Namen Dichrostachys glomerata nach einem der vorausgehenden Ansprüche, bei der das besagte Arzneimittel für einen erwachsenen Patienten in Form von Einnahmen vorliegt, die die Verabreichung einer täglichen Dosis des besagten Extrakts von 30 Gramm, verteilt auf drei Einnahmen, ermöglichen.

8. Verwendung der Heilpflanze mit dem wissenschaftlichen Namen Dichrostachys glomerata nach Anspruch 6, bei der die Extraktdosis bei jeder Einnahme nicht 15 Gramm übersteigt und vorzugsweise gleich 10 Gramm beträgt.

9. Verwendung der Heilpflanze mit dem wissenschaftlichen Namen Dichrostachys glomerata nach einem der vorausgehenden Ansprüche, bei der das besagte Arzneimittel für einen Patienten im Kindesalter in Form von Einnahmen vorliegt, die die Verabreichung einer täglichen Dosis des besagten Extrakts von 10 Gramm, verteilt auf drei Einnahmen, ermöglichen.

10. Verwendung der Heilpflanze mit dem wissenschaftlichen Namen Dichrostachys glomerata nach Anspruch 9, bei der die Extraktdosis bei jeder Einnahme 3,33 Gramm beträgt.
